# EUROPÄISCHE PATENTSCHRIFT

(11) **EP 2 944 290 B1**
(45) Veröffentlichungstag und Bekanntmachung des Hinweises auf die Patenterteilung: **11.11.2020**
(21) Anmeldenummer: 15167696.2
(22) Anmeldetag: 13.05.2015
(51) Int. Cl.: A61F 2/50, A61F 2/60

(54) **PROTHESENHÜLLE**
PROSTHETIC SHEATH
ENVELOPPE DE PROTHÈSE

(30) Priorität: 14.05.2014 DE 102014209145
(43) Veröffentlichungstag der Anmeldung: 18.11.2015
(73) Patentinhaber: Pohlig GmbH, 83278 Traunstein (DE)
(72) Erfinder: Pohlig, Kurt, 83278 Traunstein (DE); Schäfer, Michael, 83278 Traunstein (DE); Kienzle, Christian, 83064 Raubling (DE)
(74) Vertreter: Pfenning, Meinig & Partner mbB

(56) Entgegenhaltungen:
- DE-A1-102009 051 441
- US-A- 5 133 775
- US-A1- 2007 225 824
- US-A1- 2013 226 533
- US-A1- 2013 331 949

## Beschreibung

Die Erfindung betrifft eine Prothesenhülle zur Verkleidung einer Prothese, wie z.B. einer Bein- oder Armprothese. Eine Prothesenhülle wird an die eigentliche Prothese angebracht und bedeckt dabei die funktionellen Bestandteile der Prothese. Die Erfindung betrifft auch eine Prothese mit einer Prothesenhülle sowie ein Verfahren zur Herstellung einer Prothesenhülle.

Um der eigentlichen Prothese, welche ein Körperglied ersetzt, ein möglichst natürliches und unauffälliges Aussehen zu verleihen, wird oftmals eine Prothesenhülle auf die Prothese aufgezogen. Eine derartige Prothesenhülle dient u.a. zur Nachbildung der Form des Körpergliedes und besteht meist aus einem weichen Material, wie z.B. Polyethylen(PE) oder Polyurethan(PUR)-Schaum, dass eine der Haut ähnliche Konsistenz aufweist.

Derartige im Stand der Technik bekannte Prothesenhüllen weisen eine Reihe von Nachteilen auf. Zum einen sind die Prothesenhüllen gerade im Bereich von beweglichen Elementen der Prothesen, wie'z.B. an Prothesengelenken, aufgrund der ständigen Dehnung und Stauchung des Materials sehr verschleißanfällig. Zum anderen mindert die Verwendung einer Prothesenhülle den Tragekomfort der Prothese, da der Einsatz von massivem PE oder PUR-Schaum zu einem hohen Gesamtgewicht der Prothese und einer vermehrten Wärmeentwicklung in dem Prothesenschaft führt.

Außerdem haben derartige Prothesenhüllen große Nachteile beim Sport, insbesondere beim Wassersport. Beim Einsatz der Prothese im Wasser ist unbedingt darauf zu achten, dass es möglich ist, Wasser in die Prothese einzuleiten, um ein Aufschwimmen der Prothese zu verhindern. Gleichzeitig muss das Wasser in der Prothese beim Verlassen des Wassers wieder schnell aus der Prothese abfließen können. Dies wird mit den im Stand der Technik bekannten Prothesenhüllen bisher nicht zufriedenstellend erreicht.

Die Druckschrift US 5 133 775, die den nächstliegenden Stand der Technik darstellt, offenbart eine Abdeckvorrichtung für ein künstliches Bein mit einer Abdeckschicht. Die Abdeckschicht kann dabei aus halbsteifem PU-Schaum bestehen. Die Abdeckschicht weist einen flexiblen Bereich auf, der im Bereich des Gelenks des künstlichen Beins angeordnet ist. Der flexible Bereich beinhaltet eine Vielzahl von Schlitzen.

Die Druckschrift US 2013/0331949 A1 offenbart eine Roboterhand, deren Körper mit einem flexiblen Netz überzogen ist. Das Netz ist eine vermaschte Aneinanderreihung von gleichmäßig beabstandeten und miteinander verbundenen Seitenwänden, welche eine Längsausdehnung aufweisen und zwischen sich einen ausgedehnten Hohlraum definieren.

Die Druckschrift US 2013/0226533 A1 offenbart eine austauschbare Verkleidung zur Anbringung an einer Beinprothese oder -orthese, um die äußere Erscheinung der Prothese oder Orthese zu ändern. Die Verkleidung weist eine Gerüststruktur mit Gitterstegen und Durchgangslöchern auf.

Die Druckschrift DE 10 2009 051441 A1 offenbart eine Kunstgliedhülle mit einem flexiblen Gelenkbereich. Der flexible Gelenkbereich weist einen Faltenbalgbereich auf.

Ausgehend von den im Stand der Technik bekannten Problemen ist es daher die Aufgabe der vorliegenden Erfindung eine Prothesenhülle bereitzustellen, die der Prothese ein natürliches und möglichst unauffälliges Aussehen verleiht, die einen geringen Materialverschleiß im Bereich von beweglichen Prothesenelementen zeigt, die leicht ist, die eine Wärmeabfuhr aus der Prothese ermöglicht und die eine Ein- und Ausleitung von Wasser bei Verwendung der Prothesenhülle im Wasser verbessert. Ferner ist es die Aufgabe der Erfindung eine Prothese mit einer entsprechenden Prothesenhülle und ein Verfahren zur Herstellung einer entsprechenden Prothesenhülle bereitzustellen.

Die Aufgabe wird durch eine Prothesenhülle nach Anspruch 1, eine Prothese nach Anspruch 11 und ein Verfahren nach Anspruch 14 gelöst. Vorteilhafte Ausgestaltungen der Prothesenhülle und der Prothese sind in den abhängigen Ansprüchen 2 bis 10 bzw. 12 und 13 aufgeführt.

Die Prothesenhülle, die zur Verkleidung einer Prothese an die Prothese anbringbar ist, weist zumindest in einem Bereich eines beweglichen Elements der Prothese eine Gerüststruktur auf. Dabei weist die Gerüststruktur eine Mehrzahl von Gitterstegen und Durchgangsöffnungen auf, wobei die Gitterstege die Durchgangsöffnungen voneinander abgrenzen. Die Gerüststruktur erstreckt sich im Wesentlichen entlang des Umfangs und der Längsrichtung der Prothesenhülle.

Erfindungsgemäß ist in die Gerüststruktur bereichsweise eine Wandung zur partiellen Verstärkung der Gerüststruktur integriert.

Die vorliegende Erfindung ermöglicht so eine Verringerung des Gewichts der Prothesenhülle durch Einsparung von Material in Bereichen der Gerüststruktur. Aufgrund der Durchgangsöffnungen der Gerüststruktur ist die erfindungsgemäße Prothesenhülle im Bereich von beweglichen Elementen der Prothese flexibler, wodurch es weniger zur Stauchung, Quetschung und Dehnung der Prothesenhülle und somit zu einem geringeren Materialverschleiß kommt. Ferner kann über die Durchgangsöffnungen der Gerüststruktur Wärme aus der Prothese abgeführt werden. Bei der Verwendung der Prothesenhülle im Wasser kann außerdem Wasser durch die Durchgangsöffnungen schnell in die Prothese ein- und aus der Prothese heraus geleitet werden.

Ferner kann die Prothesenhülle zusätzlich auch in einem Bereich eines starren Elements die Gerüststruktur aufweisen.

Die Anordnung der Gerüststruktur hat Einfluss auf die Gewichtsverteilung der gesamten Prothese, an welcher die Prothesenhülle angebracht ist. Somit kann die Integration einer Gerüststruktur auch zu einer Optimierung der Gewichtsverteilung der gesamten Prothese dienen. So ist es vorteilhaft, in distalen Bereichen der Prothese, also in Bereichen, die entfernt von der Körpermitte liegen, die Gerüststruktur zu integrieren, da aufgrund der Gewichtsersparnis im distalen Bereich die Handhabung der Prothese durch verbesserte Hebelverhältnisse effektiv erleichtert wird.

Vorzugsweise können die Größe und/oder die Form der Durchgangsöffnungen und/oder der Gitterstege innerhalb der Bereiche, in denen die Gerüststruktur angeordnet ist, oder zwischen den Bereichen variieren. Hierdurch können die Flexibilität und Stabilität der Prothesenhülle in verschiedenen Bereichen gezielt angepasst und eingestellt werden. Je größer dabei die Durchgangsöffnungen sind, desto flexibler und nachgiebiger wird die Prothesenhülle an der entsprechenden Stelle.

Die Gerüststruktur kann netzartig, wabenförmig, lamellenförmig, spiralförmig, und/oder als Rechteckgitter ausgebildet sein. Im Fall einer spiralförmigen Gerüststruktur sind benachbarte Windungen bzw. Abschnitte einer Spirale durch

Gitterstege miteinander verbundenen, und die Abschnitte der Spirale bilden ebenfalls Gitterstege. Es ist jedoch auch denkbar, dass die Gerüststruktur innerhalb der Bereiche oder zwischen den Bereichen variiert oder als Mischform ausgebildet ist je nachdem, welche Eigenschaften, insbesondere Steifigkeit und Flexibilität, die Prothesenhülle in den verschiedenen Bereichen aufweisen soll.

Die Gerüststruktur kann einlagig oder mehrlagig ausgebildet sein. Ist die Gerüststruktur einlagig ausgebildet, handelt es sich um eine zwei-dimensionale Gerüststruktur, also um eine Gitterstruktur, die sich in einer Ebene entlang des Umfangs und der Längsausdehnung der Prothesenhülle erstreckt.

Die mehrlagige Gerüststruktur weist zusätzliche Lagen auf, die in etwa senkrecht zur Längsausdehnung oder Längsrichtung der Prothesenhülle angeordnet sind. Benachbarte Lagen sind durch im Wesentlichen senkrecht zur Längsrichtung der Prothesenhülle gerichtete Stützstege miteinander verbunden. Bei der mehrlagigen Gerüststruktur handelt es sich somit um eine dreidimensionale Gerüststruktur, die sich sowohl in einer Ebene entlang des Umfangs und senkrecht zur Längsrichtung erstreckt.

Die Gerüststruktur kann eine Dicke zwischen 0,1 cm und 2 cm, insbesondere 0,5 bis 1 cm betragen.

Ferner kann die Dicke der Gerüststruktur in verschiedenen Bereichen der Prothese unterschiedlich sein. Hierbei können eine Tiefe der Gitterstege und/oder eine Anzahl von Lagen der Gerüststruktur innerhalb der Bereiche oder zwischen den Bereichen mit der Gerüststruktur, also von Bereich zu Bereich, variieren. Hierdurch lässt sich die Flexibilität und Stabilität der Prothesenhülle ebenfalls gezielt an die Prothese anpassen und einstellen. So kann in Bereichen der Prothesenhülle, in denen eine hohe Stabilität und Festigkeit und gleichzeitig eine begrenzte Flexibilität der Prothesenhülle erforderlich ist, die Gerüststruktur mehrlagig ausgebildet sein. Tiefere Gitterstege verringern die Flexibilität und erhöhen die Steifigkeit der Prothesenhülle.

In einer weiteren vorteilhaften Variante weist die Prothesenhülle mehrheitlich oder vollständig die Gerüststruktur auf oder besteht daraus. Eine derart ausgebildete Prothesenhülle eignet sich insbesondere für den Einsatz beim Schwimmen, da durch die großflächige Gerüststruktur schnell Wasser ein- und ausfließen kann.

Vorteilhafterweise ist die Prothesenhülle derart ausgebildet, dass sie die Prothese weitgehend umschließt, also weitgehend verkleidet, sodass die Prothese unter der Prothesenhülle weitgehend verborgen wird. Alternativ kann die Prothesenhülle auch lediglich zur bereichsweisen Verkleidung einer Prothese ausgebildet sein, sodass beispielsweise lediglich ein Prothesengelenk verkleidet wird.

Die Prothesenhülle kann auch mehrere Teile aufweisen. Die verschiedenen Teile sind vorteilhafterweise über eine Nut-Feder-Verbindung, eine Nut-Spund-Verbindung, einen Magnetverbindung oder einen Schraubverbindung miteinander verbunden. Alternativ können die Teile auch ineinander gesteckt und/oder miteinander verklebt sein.

Die erfindungsgemäße Prothesenhülle kann zur Verkleidung aller Arten von Prothesen oder Epithesen ausgebildet sein, insbesondere zur Verkleidung einer Bein-, Arm-, Unterschenkel-, Unterarm-, Fuß- oder Handprothese ausgebildet sein. Ist die Prothesenhülle zur Verkleidung einer Unterschenkelprothese ausgebildet, kann die Prothesenhülle einen Fußteil, einen mit dem Fußteil verbundenen Gelenkteil und einen mit dem Gelenkteil verbundenen Unterschenkelteil aufweisen. Hierbei weist der Gelenkteil die Gerüststruktur auf. Vorzugsweise können auch der Fußteil und/oder der Unterschenkelteil die Gerüststruktur aufweisen.

Erfindungsgemäß ist auch eine Prothese zum Schwimmen und Laufen, die eine vorstehend beschriebene, erfindungsgemäße Prothesenhülle aufweist. Die Prothese kann einen Prothesenschaft aufweisen, auf den die Prothesenhülle aufgezogen ist. Dabei kann die Prothesenhülle in ihrem dem der Prothese zugewandten Innenbereich formschlüssig an der Prothese anliegen oder von der Prothese beabstandet sein, sodass ein Hohlraum zwischen der Prothese und der Prothesenhülle besteht. Ferner kann die Prothesenhülle durch Reibschluss am Prothesenschaft haften oder mit diesem bereichsweise verklebt sein.

Vorzugsweise weist die Prothese ein feststellbares Prothesengelenk auf. Der Mechanismus zur Feststellung des Prothesengelenks kann u.a. mechanisch, hydraulisch oder pneumatisch sein. Ein feststellbares Prothesengelenk ermöglicht es, beispielsweise dieselbe Prothese beim Laufen und Schwimmen zu verwenden. Während des Laufens ist das Prothesengelenk gelöst, wodurch eine normale Laufbewegung möglich ist. Beim Schwimmen kann das Prothesengelenk zur Verbesserung der Handhabung der Prothese im Wasser festgestellt werden.

Der Prothesenschaft kann ferner zumindest bereichsweise perforiert sein. Der perforierte Schaft ermöglicht zusammen mit einer im Bereich des Prothesenschafts integrierten Gerüststruktur der Prothesenhülle eine Belüftung eines vom Prothesenschaft aufgenommenen Amputationsstumpfes.

Die Prothesenhülle der Prothese kann auch mit einem Überzug versehen sein. Der Überzug kann ein elastisches Material und/oder Silikon enthalten oder daraus bestehen. Zur Anbringung an der Prothesenhülle kann der Überzug auf die Prothesenhülle aufgerollt sein und an der Prothesenhülle und/oder dem Prothesenschaft reibschlüssig haften. Zusätzlich kann der Überzug auch noch mit der Prothesenhülle und/oder dem Prothesenschaft bereichsweise verklebt sein.

Eine Dicke des Überzugs kann zwischen 0,05 cm und 1 cm, insbesondere 0,1 cm bis 0,5 cm, betragen.

Ferner kann eine Farbe des Überzugs an eine Hautfarbe eines durch die Prothese zu ersetzenden Körpergliedes oder an eine Hautfarbe eines zweiten, gesunden Körpergliedes angepasst sein.

Anstelle eines Überzugs kann die Prothese auch mit einer über die Prothesenhülle gezogenen Konfektionssocke verwendet werden.

Erfindungsgemäß ist auch ein Verfahren zur Herstellung einer vorstehend beschriebenen Prothesenhülle. Hierbei wird zur Modellierung einer Form des zu ersetzenden Körpergliedes eine Form eines gesunden Körperglieds gespiegelt und mittels eines Rapid-Prototyping-Verfahrens, mittels eines Spritzgußverfahrens oder mittels Fräsens die Prothesenhülle oder Teile davon hergestellt, welche die gespiegelte Form des gesunden Körperteils aufweisen. Unter Spiegeln einer Form eines gesunden Körpergliedes wird hier die Erzeugung einer seitenverkehrten Abbildung der Form eines gesunden Körpergliedes, bspw. eines gesunden linken Unterschenkels, verstanden, um auf Basis dieser seitenverkehrten Abbildung die Form des korrespondierenden zu ersetzenden Körperteils, bspw. eines zu ersetzenden rechten Unterschenkels des Patienten, zu modellieren. Bei der Herstellung der Prothesenhülle durch Fräsen wird die Prothesenhülle oder Teile davon in einen Materialblock eingefräst.

Die Prothesenhülle oder die Teile der Prothesenhülle werden bevorzugt einstückig gefertigt.

Die Prothesenhülle weist insgesamt ein elastisches und/oder flexibles Material auf, dessen Konsistenz vorzugsweise dem durch die Prothese zu ersetzenden Körperteil nachempfundenen ist. Wird die Prothesenhülle mittels des Rapid-Prototyping-Verfahrens hergestellt, weist die Prothesenhülle vorzugsweise Polyamid (PA), Nylon, Photopolymere, Polylactide (PLA), Acrylnitril-Butadien-Styrol (ABS), Polycarbonat (PC), Polyvinylchlorid(PVC), Polyurethan (PU) und/oder thermoplastisches Polyurethan (TPU) auf oder besteht daraus. Bei der Anwendung anderer Herstellungsverfahren, beispielsweise dem Spritzgußverfahren, werden vorzugsweise Elastomere, Duroplaste oder ähnliche Materialien für die Prothesenhülle verwendet.

Im Folgenden werden einige Beispiele einer erfindungsgemäßen Prothesenhülle und einer erfindungsgemäßen Prothese anhand von Figuren beschrieben. Dabei werden verschiedene erfindungswesentliche oder vorteilhafte Elemente im Rahmen dieser Beispiele genannt, wobei auch einzelne dieser Elemente als solche zur Weiterbildung der Erfindung - auch herausgelöst aus dem Kontext der Beispiele - verwendet werden können. Ferner entsprechen in den Figuren gezeigte gleiche oder ähnliche Elemente gleichen oder ähnlichen Bezugszeichen.

### Es zeigen

- Figur 1: eine Seitenansicht einer beispielhaften Prothesenhülle für eine Unterschenkelprothese,
- Figur 2: eine Schnittansicht des Unterschenkelteils aus Figur 1 im Bereich des Prothesenschafts,
- Figur 3: eine Seitenansicht einer weiteren beispielhaften Prothesenhülle für eine Unterschenkelprothese,
- Figur 4: eine Detailansicht des Gelenkteils der Prothesenhülle aus Figur 3,
- Figur 5: eine Schnittansicht einer Verbindung zwischen dem Gelenkteil und dem Unterschenkelteil der Prothesenhülle aus Figur 3,
- Figur 6: eine Detailansicht einer Verbindung zwischen dem Fußteil und dem Gelenkteil der Prothesenhülle aus Figur 3,
- Figur 7: eine weitere Seitenansicht der Prothesenhülle aus Figur 3,
- Figur 8: eine weitere Seitenansicht der Prothesenhülle aus Figur 3,
- Figur 9: eine Seitenansicht einer erfindungsgemäßen Prothesenhülle für eine Unterschenkelprothese,
- Figur 10: eine Seitenansicht einer weiteren erfindungsgemäßen Prothesenhülle für eine Unterschenkelprothese,
- Figur 11: eine Seitenansicht einer erfindungsgemäßen Unterschenkelprothese mit Prothesenhülle und
- Figur 12: eine Seitenansicht eines Prothesenfußes einer erfindungsgemäßen Unterschenkelprothese mit Prothesenhülle.

Es werden die folgenden Bezugszeichen verwendet:
- 1: Prothesenhülle
- 2: Unterschenkelteil
- 3: Gelenkteil
- 4: Fußteil
- 5a: Gitterstege Unterschenkelteil
- 5b: Gitterstege Gelenkteil
- 5c: Gitterstege Fußteil
- 6a: Durchgangslöcher Unterschenkelteil
- 6b: Durchgangslöcher Gelenkteil
- 6c: Durchgangslöcher Fußteil
- 7: Verbindung Unterschenkelteil-Gelenkteil
- 7a: Erstes Verbindungsteil
- 7b: Zweites Verbindungsteil
- 8: Verbindung Gelenkteil-Fußteil
- 8a: Spund
- 8b: Nut
- 9: Prothesenschaft
- 10: Wandung
- 11: Prothesenfuß
- 12: Prothesenunterschenkel
- 13: Abschlussbereich
- 14: Haftbereich

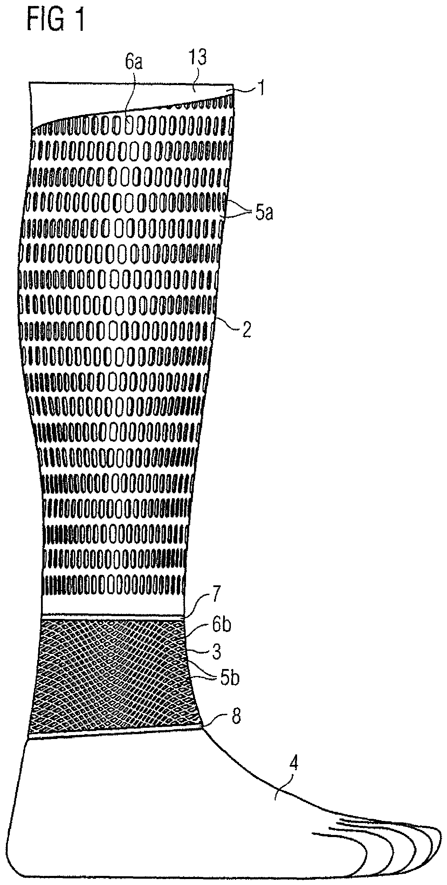

Die in den Figuren 1 bis 8 dargestellten beispielhaften Prothesenhüllen sind nicht Teil der Erfindung. Die Darstellung und Beschreibung dieser Beispiele ist jedoch hilfreich für das Verständnis der Erfindung.

Figur 1 zeigt eine perspektivische Seitenansicht einer beispielhaften Prothesenhülle 1 für eine rechte Unterschenkelprothese. Die Prothesenhülle 1 weist die äußere Form eines Unterschenkels unterhalb des Kniegelenks auf.

Die Prothesenhülle 1 weist einen im Wesentlichen zylinderförmigen Unterschenkelteil 2 auf, der sich in etwa von unterhalb des Knies bis zum Sprunggelenk erstreckt und eine künstliche Wade bildet. Im Bereich des Sprunggelenks schließt sich formschlüssig ein Gelenkteil 3 an den Unterschenkelteil 2 an. Der Gelenkteil 3 weist eine Form auf, die dem körperlichen Bereich des Sprunggelenks nachempfunden ist.

Der Unterschenkelteil 2 und der Gelenkteil 3 sind über eine formschlüssige Verbindung 7 miteinander verbunden. An den Gelenkteil 3 schließt sich unterhalb des Sprunggelenks ein Fußteil 4 an. Der Fußteil 4 weist eine Form auf, die in etwa dem Fuß nachempfunden ist. Der Gelenkteil 3 und der Fußteil 4 sind über eine formschlüssige Verbindung 8 miteinander verbunden.

Der Unterschenkelteil 2 und der Gelenkteil 3 weisen jeweils verschiedene Gerüststrukturen auf, die sich im Wesentlichen bis auf Bereiche um die Verbindungen 7 und 8 und dem dem Gelenkteil 3 abgewandten Abschlussbereich 13 des Unterschenkelteils 2 in einer Ebene entlang des gesamten Umfangs des Unterschenkelteils 2 und des Gelenkteils 3 erstrecken. Die Gerüststruktur des Unterschenkelteils 2 weist ovale Durchgangslöcher 6a auf, deren Längsachse parallel zur Längsachse der Prothesenhülle 1 ausgerichtet ist und die durch im Wesentlichen senkrecht zueinander verlaufende Gitterstege 5a voneinander getrennt sind. Die Gitterstege 5a verlaufen im Wesentlichen parallel zur Längsachse der Prothesenhülle 1 und parallel zum Umfang der Prothesenhülle 1. Die Tiefe der Gitterstege 5a radial zur Längsachse der Prothesenhülle 1, und damit auch die Dicke des Unterschenkelteils 2, entspricht im Wesentlichen der Breite der Durchgangslöcher 6a. Das Verhältnis von Länge zu Breite der Durchgangslöcher 6a beträgt in etwa zwei zu eins. Das Verhältnis der Breite der in Umfangsrichtung verlaufenden Gitterstege 5a zur Breite der in Längsrichtung verlaufenden Gitterstege 5a ist ebenfalls in etwa zwei zu eins. In der dem Gelenkteil 3 zugewandten unteren Hälfte des Unterschenkelteils 2 nimmt die Breite der Durchgangslöcher 6a sowie die Breite der in Längsrichtung verlaufenden Gitterstege 5a ab. Die Durchgangslöcher 6a sind entlang des Umfangs in Reihe und in Längsrichtung versetzt zueinander angeordnet.

Der Gelenkteil 3 weist eine wabenförmige Gerüststruktur mit Durchgangslöchern 6b und die Durchgangslöcher 6b begrenzende Gitterstege 5b auf. Die Durchgangslöcher 6b sind sechs-kantig mit gleichen Kantenlängen geformt. Die Breite der Durchgangslöcher 6b entspricht in etwa der Tiefe der Gitterstege 5b und somit in etwa der Dicke des Gelenkteils 3. Die Breite der Durchgangslöcher 6b entspricht in etwa der Breite der Durchgangslöcher 6a im dem Gelenkteil 3 abgewandten oberen Bereich des Unterschenkelteils 2.

Der Fußteil 4 weist eine komplett geschlossene Struktur und somit keine Gerüststruktur auf.

Der Unterschenkelteil 2, der Gelenkteil 3 und der Fußteil 4 können sowohl größtenteils hohl als auch massiv ausgebildet sein. Sind sie hohl ausgebildet liegt die Prothesenhülle 1 größtenteils nicht an der Prothese an. Zwischen Prothese und Prothesenhülle 1 befindet sich vielmehr ein Hohlraum. Ist die Prothesenhülle 1 dagegen massiv ausgebildet, kann die Prothesenhülle 1 in ihrem der Prothese zugewandten Innenbereich formschlüssig an der Prothese anliegen. Die in Figur 1 gezeigte Prothesenhülle 1 hätte dann auch im Innenbereich des Unterschenkelteils 2 und des Gelenkteils 3 eine Gerüststruktur, die entweder mehrlagig ausgebildet ist oder deren Gitterstege 5a, 5b eine entsprechende Tiefe aufweisen, sodass die Gitterstege bis an die eigentliche Prothese heranreichen.

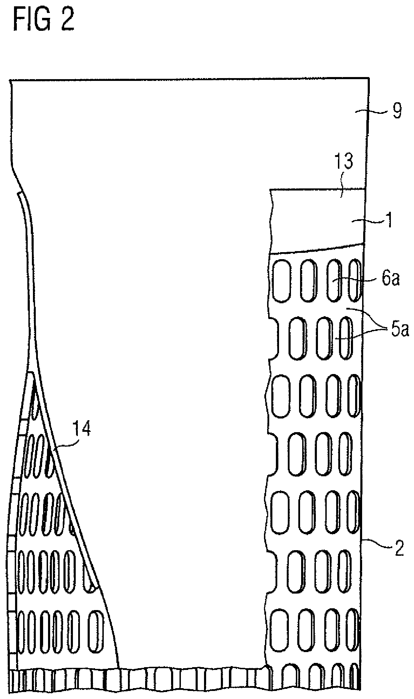

Figur 2 zeigt eine perspektivische Schnittansicht eines oberen Bereichs des Unterschenkelteils 2 der Prothesenhülle aus Figur 1, wobei das Unterschenkelteil 2 auf einen Prothesenschaft 9 aufgezogen ist. Zur Ansicht der dem Prothesenschaft 9 zugewandten Innenseite des Unterschenkelteils 2 ist aus dem Unterschenkelteil 2 ein rechteckiger Abschnitt ausgeschnitten. Der obere Bereich des Unterschenkelteils 2 weist einen Abschlussbereich 13 auf, der form- und reibschlüssig am Prothesenschaft 9 anliegt. In distaler Richtung (Richtung Fußteil) zweigt vom Unterschenkelteil 2 ein flächiger Haftbereich 14 ab, der weiter eng am Prothesenschaft 9 reib- und fromschlüssig anliegt, während das eigentliche Unterschenkelteil 2 der Prothesenhülle 1 vom Prothesenschaft 9 beabstandet ist, um die Wölbung des Wadenmuskels nachzubilden. Der Haftbereich 14 erstreckt sich entlang des Umfangs des Prothesenschafts 9 und in Längsrichtung distal in etwa bis auf Höhe der nachgebildeten Wadenmuskelwölbung. Der Haftbereich 14 verhindert ein Abrutschen und Verdrehen des Unterschenkelteils 2 und somit der gesamten Prothesenhülle 1.

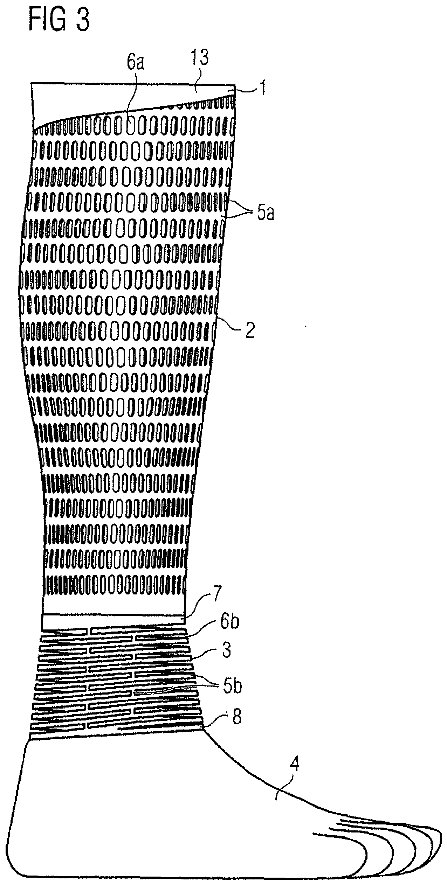

Figur 3 zeigt eine perspektivische Seitenansicht einer ähnlichen Prothesenhülle 1 wie in Figur 1, wobei der Gelenkteil 3 in Figur 3 nun eine spiralförmige Gerüststruktur aufweist.

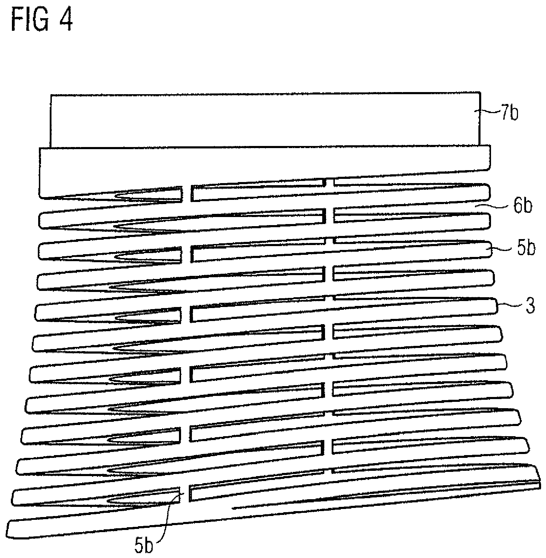

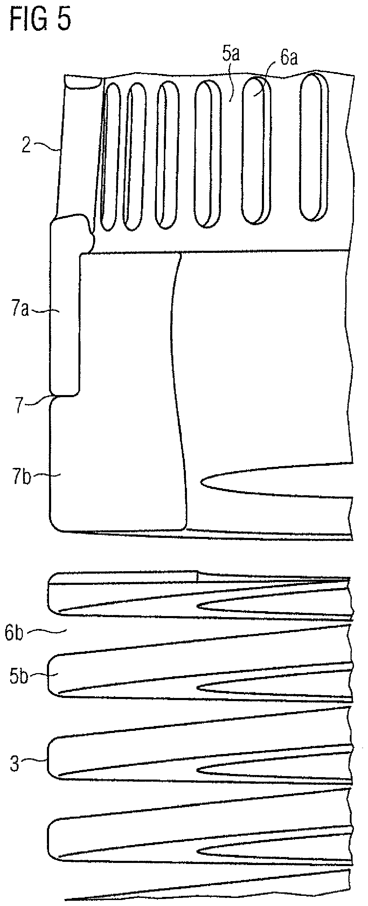

Die spiralförmige Gerüststruktur des Gelenkteils 3 aus Figur 3 ist in Figur 4 im Detail dargestellt. Figur 4 zeigt den oberen, dem Unterschenkelteil 2 zugewandten Teil des Gelenkteils 3 in einer perspektivischen Ansicht. Dieser weist an seinem oberen Ende ein verjüngtes zweites Verbindungsteil 7b auf, das in das erste Verbindungsteil 7a des Unterschenkelteils 2 zur Bildung der Verbindung 7 gesteckt wird (siehe Figur 5). An das zweite Verbindungsteil 7b schließt sich in distaler Richtung die spiralförmige Gerüststruktur an. Dabei bilden Abschnitte der Spirale selbst und die benachbarte Windungen miteinander verbindenden Stege die Gitterstege 5b. Die Durchgangslöcher 6b werden von den Gitterstegen 5b, also den benachbarten Windungsabschnitten oder Spiralabschnitten und den Verbindungsstegen begrenzt.

Figur 5 zeigt die Verbindung 7 zwischen dem Unterschenkelteil 2 und dem Gelenkteil 3 der Prothesenhülle 1 aus Figur 3 in einer perspektivischen Schnittansicht. An seinem dem Gelenkteil 3 zugewandten Ende weist der Unterschenkelteil 2 ein erstes Verbindungsteil 7a zur Aufnahme des zweiten Verbindungsteiles 7b auf. Das erste Verbindungsteil 7a ist auf das zweite Verbindungsteil 7b gesteckt, sodass die formschlüssige Verbindung 7 gebildet wird.

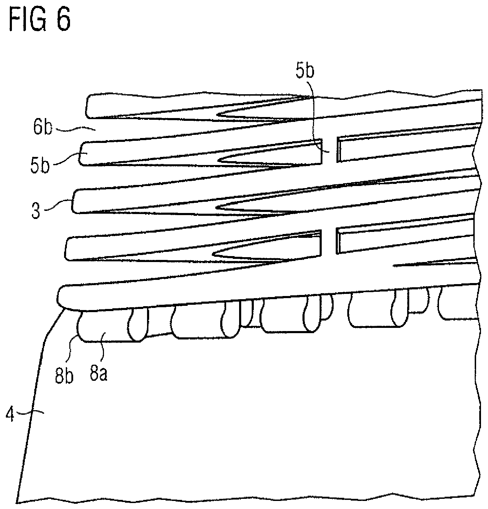

Figur 6 zeigt eine transparente, perspektivische Ansicht der Verbindung 8 zwischen dem Gelenkteil 3 und dem Fußteil 4 der Prothesenhülle 1 aus Figur 3. Der Gelenkteil 3 weist an seinem dem Fußteil 4 zugewandten Ende Spunde 8a auf, welche in eine Nut 8b des dem Gelenkteil 3 zugewandten Ende des Fußteils 4 gesteckt sind, um eine formschlüssige Nut-Spund-Verbindung zu bilden.

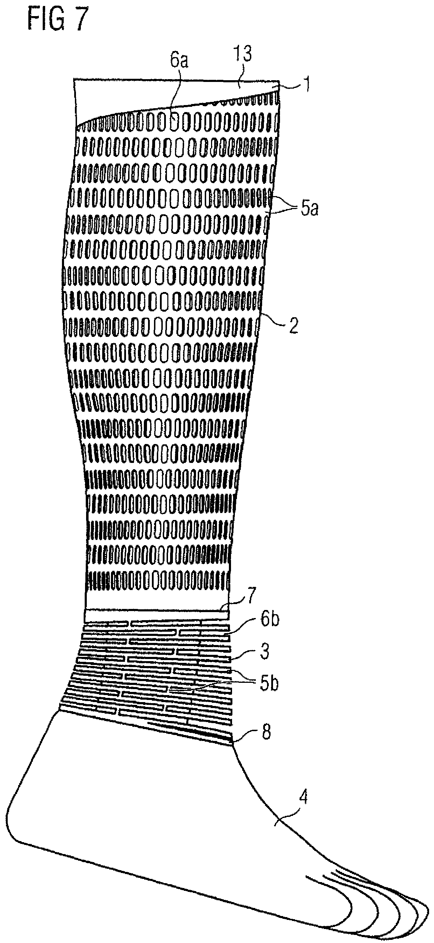

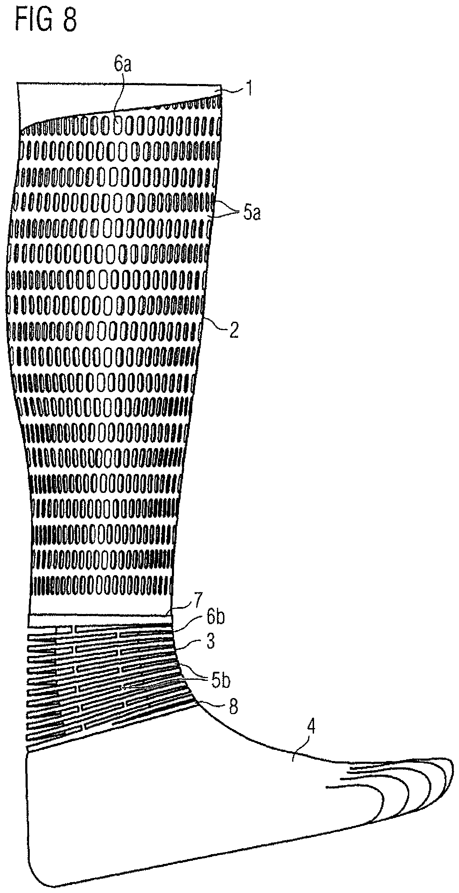

Figuren 7 und 8 zeigen die Funktion der Gerüststruktur des Gelenkteils 3 der Prothesenhülle 1 aus Figur 3. Aufgrund der Gerüststruktur ist es möglich, das Fußteil 4 zum Unterschenkelteil 2 hin zu ziehen bzw. das Fußteil 4 vom Unterschenkelteil 2 weg zu strecken, ohne dass es zu einer Stauchung oder Dehnung des Gelenkteils 3 der Prothesenhülle 1 kommt.

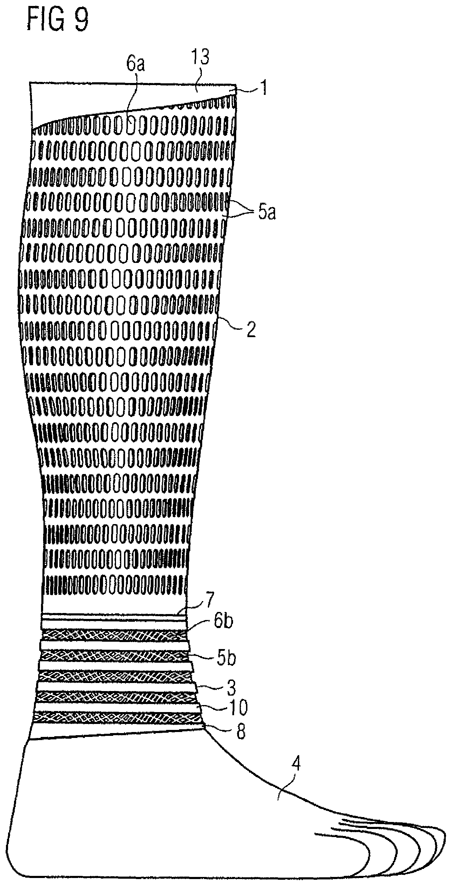

Figur 9 zeigt eine erfindungsgemäße Prothesenhülle 1, die eine ähnliche Prothesenhülle 1 wie in Figur 3 ist, wobei der Gelenkteil 3 der Prothesenhülle 1 in Figur 9 bereichsweise eine zusätzliche Wandung 10 aufweist, welche die Gerüststruktur des Gelenkteils 3 überdeckt. Die Wandung 10 ist in ringförmigen Streifen in gleichen Abständen in Längsrichtung der Prothesenhülle zylinderförmig am Gelenkteil 3 angeordnet und dient zur Stabilisierung des Gelenkteils 3 bei gleichzeitig hoher Flexibilität des Gelenkteils 3.

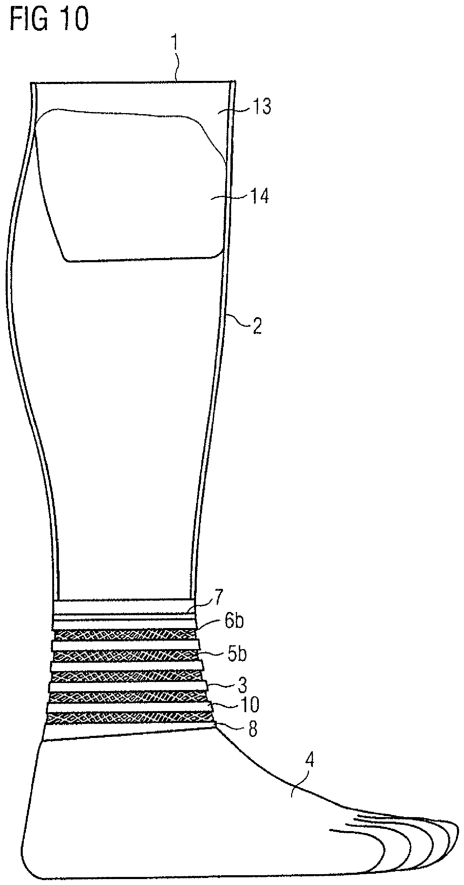

Figur 10 zeigt eine ähnliche Prothesenhülle wie in Figur 9 in einer transparenten Seitenansicht, wobei das Unterschenkelteil 2 nun eine geschlossene Struktur, also keine Gerüststruktur, aufweist. Am dem Gelenkteil 3 abgewandten Ende des Unterschenkteils 2 ist der Abschlussbereich 13 angeordnet, der nach unten hin in den Haftbereich 14 im Innenbereich der Prothesenhülle 1 und den eigentlichen, von außen sichtbaren Unterschenkelteil 2 übergeht.

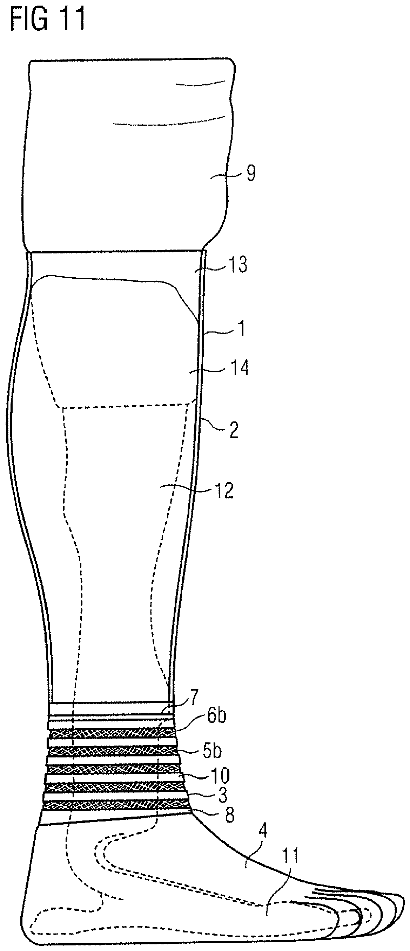

Figur 11 zeigt eine transparente, perspektivische Seitenansicht einer Prothese mit der Prothesenhülle 1 aus Figur 10. Die Prothesenhülle 1 ist auf die Prothese aufgezogen, die einen Prothesenschaft 9, einen Prothesenunterschenkel 12 und einen Prothesenfuß 11 aufweist. Die Prothesenhülle 1 haftet mit ihrem Abschlussbereich 13 und ihrem Haftbereich 14 am Prothesenschaft 9.

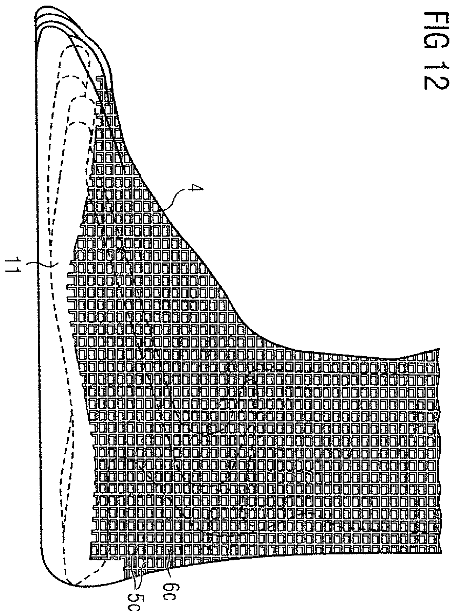

Figur 12 zeigt eine transparente, perspektivische Seitenansicht eines linken Prothesenfußes 11 mit einem Fußteil 4 einer Prothesenhülle 1 für eine linke Unterschenkelprothese. Der Fußteil 4 weist bis auf den Sohlen-, Zehen- und Fersenbereich überwiegend eine Gerüststruktur auf. Die Durchgangslöcher 6c sind rechteckig und werden von senkrecht zueinander verlaufenden Gitterstegen 5c begrenzt.

## Patentansprüche

1. Prothesenhülle (1) zur Verkleidung einer Prothese, wobei die Prothesenhülle (1) an einer Prothese anbringbar ist und
zumindest in einem Bereich eines beweglichen Elements der Prothese eine Gerüststruktur mit einer Mehrzahl von Gitterstegen (5a-c) und Durchgangsöffnungen (6a-c) aufweist, wobei die Gitterstege (5a-c) die Durchgangsöffnungen (6a-c) voneinander abgrenzen,
**dadurch gekennzeichnet, dass**
in die Gerüststruktur bereichsweise eine Wandung (10) zur partiellen Verstärkung der Gerüststruktur integriert ist.

2. Prothesenhülle (1) nach dem vorhergehenden Anspruch, **dadurch gekennzeichnet, dass** die Prothesenhülle (1) in einem Bereich eines starren Elements der Prothese die Gerüststruktur aufweist.

3. Prothesenhülle nach dem vorhergehenden Anspruch, **dadurch gekennzeichnet, dass** eine Größe und/oder Form der Durchgangsöffnungen (6a-c) und/oder der Gitterstege (5a-c) innerhalb der Bereiche oder zwischen den Bereichen variiert.

4. Prothesenhülle (1) nach einem der vorhergehenden Ansprüche, **dadurch gekennzeichnet, dass** die Gerüststruktur einlagig ausgebildet ist oder mehrlagig ausgebildet ist, wobei benachbarte Lagen durch Stützstege miteinander verbunden sind.

5. Prothesenhülle (1) nach einem der vorhergehenden Ansprüche, **dadurch gekennzeichnet, dass** eine Tiefe der Gitterstege (5a-c) und/oder eine Anzahl von Lagen der Gerüststruktur in den Bereichen oder zwischen den Bereichen variiert.

6. Prothesenhülle (1) nach einem der vorhergehenden Ansprüche, **dadurch gekennzeichnet, dass** die Prothesenhülle (1) mehrere Teile (2, 3, 4) aufweist, wobei die Teile (2, 3, 4) über eine Nut-Feder-Verbindung, eine Nut-Spund-Verbindung (8), eine Magnetverbindung oder eine Schraubverbindung miteinander verbunden, ineinander gesteckt und/oder verklebt sind.

7. Prothesenhülle (1) nach einem der vorhergehenden Ansprüche, **dadurch gekennzeichnet, dass** die Prothesenhülle (1) elastisch ist, eine einem durch die Prothese zu ersetzenden Körperglied nachempfundene Konsistenz aufweist und/oder ein flexibles Material, Polyamid, Nylon, Photopolymere, Polylactide, Acrylnitril-Butadien-Styrol, Polycarbonat, Polyvinylchlorid, Polyurethan, Elastomere und/oder Duroplaste enthält oder daraus besteht.

8. Prothesenhülle (1) nach einem der vorhergehenden Ansprüche, **dadurch gekennzeichnet, dass** die Prothesenhülle (1) zur Verkleidung einer Bein-, Arm-, Unterschenkel-, Unterarm-, Fuß- oder Handprothese oder einer Epithese ausgebildet ist.

9. Prothesenhülle (1) nach einem der vorhergehenden Ansprüche zur Verkleidung einer Unterschenkelprothese, wobei die Prothesenhülle (1) einen Fußteil (4), einen mit dem Fußteil (4) verbundenen Gelenkteil (3) und einen mit dem Gelenkteil (3) verbundenen Unterschenkelteil (2) aufweist, und wobei der Gelenkteil (3) die Gerüststruktur aufweist.

10. Prothesenhülle (1) nach dem vorhergehenden Anspruch, **dadurch gekennzeichnet, dass** der Fußteil (4) und/oder der Unterschenkelteil (2) die Gerüststruktur aufweisen.

11. Prothese zum Schwimmen und Laufen, **gekennzeichnet durch** eine Prothesenhülle (1) nach einem der Ansprüche 1 bis 10.

12. Prothese nach dem vorhergehenden Anspruch, **dadurch gekennzeichnet, dass** die Prothesenhülle (1) mit einem Überzug versehen ist.

13. Prothese nach dem vorhergehenden Anspruch, **dadurch gekennzeichnet, dass** der Überzug ein elastisches Material und/oder Silikon enthält oder daraus besteht.

14. Verfahren zur Herstellung einer Prothesenhülle (1) nach einem der Ansprüche 1 bis 10, **dadurch gekennzeichnet, dass** zur Modellierung einer Form des zu ersetzenden Körpergliedes eine Form eines gesunden Körpergliedes gespiegelt wird und mittels eines Rapid-Prototyping-Verfahrens, mittels eines Spritzgußverfahrens oder mittels Fräsens die Prothesenhülle (1) hergestellt wird, welche die gespiegelte Form des gesunden Körperteils aufweist.

## Claims

1. A prosthesis sheath (1) to encase a prosthesis, wherein the prosthesis sheath (1) can be fitted onto a prosthesis and at least in a region of a movable element of the prosthesis has a framework structure with a plurality of grid strips (5a-c) and through openings (6a-c), wherein the grid strips (5a-c) separate the through openings (6a-c) from one another, **characterised in that** a wall (10) for partial reinforcement of the framework structure is integrated into regions of the framework structure.

2. A prosthesis sheath (1) according to the preceding claim, **characterised in that** the prosthesis sheath (1) has the framework structure in a region of a rigid element of the prosthesis.

3. A prosthesis sheath according to the preceding claim, **characterised in that** a size and/or form of the through openings (6a-c) and/or the framework strips (5a-c) varies within the regions or between the regions.

4. A prosthesis sheath (1) according to any one of the preceding claims, **characterised in that** the framework structure is single-layered or multi-layered, wherein adjacent layers are connected to one another by support strips.

5. A prosthesis sheath (1) according to any one of the preceding claims, **characterised in that** a depth of the framework strips (5a-c) and/or a number of layers of the framework structure varies/vary in the regions or between the regions.

6. A prosthesis sheath (1) according to any one of the preceding claims, **characterised in that** the prosthesis sheath (1) has a plurality of parts (2, 3, 4), wherein the parts (2, 3, 4) are connected to one another via a tongue-in-groove connection, a tongue-and-groove connection (8), a magnetic connection or a screw connection, are inserted into one another and/or are stuck to one another.

7. A prosthesis sheath (1) according to any one of the preceding claims, **characterised in that** the prosthesis sheath (1) is elastic, has a consistency modeled on the body member to be replaced by the prosthesis and/or comprises a flexible material, polyamide, nylon, photopolymers, polylactides, acrylonitrile butadiene styrene, polycarbonate, polyvinyl chloride, polyurethane, elastomers and/or thermosetting plastics or consists thereof.

8. A prosthesis sheath (1) according to any one of the preceding claims, **characterised in that** the prosthesis sheath (1) is formed to encase a leg prosthesis, arm prosthesis, lower leg prosthesis, forearm prosthesis, foot prosthesis or hand prosthesis or an epithesis.

9. A prosthesis sheath (1) according to any one of the preceding claims for encasing a lower leg prosthesis, wherein the prosthesis sheath (1) has a foot part (4), an ankle part (3) connected to the foot part (4) and a lower leg part (2) connected to the ankle part (3), and wherein the ankle part (3) has the framework structure.

10. A prosthesis sheath (1) according to the preceding claim, **characterised in that** the foot part (4) and/or the lower leg part (2) have the framework structure.

11. A prosthesis for swimming and running, **characterised by** a prosthesis sheath (1) according to any one of claims 1 to 10.

12. A prosthesis according to the preceding claim, **characterised in that** the prosthesis sheath (1) is provided with a cover.

13. A prosthesis according to the preceding claim, **characterised in that** the cover comprises an elastic material and/or silicone or consists thereof.

14. A method of manufacturing a prosthesis sheath (1) according to any one of claims 1 to 10, **characterised in that** a form of a healthy body member is mirrored for modelling of a form of the body member to be replaced and the prosthesis sheath (1) which has the mirrored form of the healthy body part is manufactured by means of a rapid prototyping process, by means of an injection moulding process or by means of milling.

## Revendications

1. Gaine de prothèse (1) pour le revêtement d'une prothèse, dans laquelle la gaine de prothèse (1) peut être appliquée sur une prothèse et
qui comprend, au moins dans une zone d'un élément mobile de la prothèse, une structure d'armature avec une pluralité de traverses de grilles (5a-c) et d'ouvertures de passage (6a-c), dans laquelle les traverses de grilles (5a-c) séparent les ouvertures de passage (6a-c) les unes des autres,
**caractérisée en ce que**
dans la structure d'armature est intégrée, dans certaines zones, une paroi (10) pour le renforcement partiel de la structure d'armature.

2. Gaine de prothèse (1) selon la revendication précédente, **caractérisée en ce que** la gaine de prothèse (1) présente la structure d'armature dans une zone d'un élément rigide de la prothèse.

3. Gaine de prothèse selon la revendication précédente, **caractérisée en ce qu'**une taille et/ou une forme des ouvertures de passage (6a-c) et/ou des traverses de grille (5a-c) varie à l'intérieur des zones ou entre les zones.

4. Gaine de prothèse (1) selon l'une des revendications précédentes, **caractérisée en ce que** la structure d'armature est constituée d'une seule couche ou de plusieurs couches, dans laquelle les couches adjacentes sont reliées entre elles par des traverses de soutien.

5. Gaine de prothèse (1) selon l'une des revendications précédentes, **caractérisée en ce qu'**une profondeur des traverses de grille (5a-c) et/ou un nombre de couches de la structure d'armature varie dans les zones ou entre les zones.

6. Gaine de prothèse (1) selon l'une des revendications précédentes, **caractérisée en ce que** la gaine de prothèse (1) comprend plusieurs parties (2, 3, 4), dans laquelle les parties (2, 3, 4) sont reliées, emboîtées et/ou collées entre elles par l'intermédiaire d'une liaison par rainure et languette, une liaison par rainure et bouchon (8), une liaison magnétique ou une liaison vissée.

7. Gaine de prothèse (1) selon l'une des revendications précédentes, **caractérisée en ce que** la gaine de prothèse (1) est élastique, **en ce qu'**elle présente une consistance proche de celle du membre remplacé par la prothèse et/ou contient ou est constituée d'un matériau flexible, de polyamide, de nylon, de photopolymères, de polylactides, d'acrylnitrile-butadiène-styrène, de polycabonate, de polychlorure de vinyle, de polyuréthane, d'élastomères et/ou de matières plastiques thermodurcissables.

8. Gaine de prothèse (1) selon l'une des revendications précédentes, **caractérisée en ce que** la gaine de prothèse (1) est conçue pour le revêtement d'une prothèse de jambe, de bras, de mollet, d'avant-bras, de pied ou de main ou d'une épithèse.

9. Gaine de prothèse (1) selon l'une des revendications précédentes, pour le revêtement d'une prothèse de mollet, dans laquelle la gaine de prothèse (1) comprend une partie de pied (4), une partie d'articulation (3) reliée avec la partie de pied (3) et une partie de mollet (2) reliée avec la partie d'articulation (3) et dans laquelle la partie d'articulation (3) comprend la structure d'armature.

10. Gaine de prothèse (1) selon la revendication précédente, **caractérisée en ce que** la partie de pied (4) et/ou la partie de mollet (2) comprennent la structure d'armature.

11. Prothèse pour la natation et la course, **caractérisée par** la présence d'une gaine de prothèse (1) selon l'une des revendications 1 à 10.

12. Prothèse selon la revendication précédente, **caractérisée en ce que** la gaine de prothèse (1) est munie d'un revêtement.

13. Prothèse selon la revendication précédente, **caractérisée en ce que** le revêtement contient ou est constitué d'un matériau élastique et/ou de silicone.

14. Procédé de fabrication d'une gaine de prothèse (1) selon l'une des revendications 1 à 10, **caractérisé en ce que**, pour la modélisation d'une forme du membre à remplacer, une forme d'un membre sain est mise en miroir et, au moyen d'un procédé de prototypage rapide, au moyen d'un procédé de moulage par injection ou au moyen d'un fraisage, la gaine de prothèse (1) est fabriquée, qui présente la forme en miroir de la partie saine du corps.
